# FASCICULE DE BREVET EUROPEEN

(11) **EP 1 489 970 B1**
(45) Date de publication et mention de la délivrance du brevet: **26.07.2006**
(21) Numéro de dépôt: 03738206.6
(22) Date de dépôt: 27.03.2003
(51) Int. Cl.: A61B 7/02, A61B 8/06

(54) **APPAREIL DE DEPISTAGE ET DE DIAGNOSTIC PAR DOUBLE DETECTION STETHOSCOPIQUE ET DOPPLER**
GERÄT ZUR UNTERSUCHUNG UND DIAGNOSE DURCH DOPPELSTETHOSKOPISCHEN UND DOPPLER-NACHWEIS
APPARATUS FOR SCREENING AND DIAGNOSING BY DUAL STETHOSCOPIC AND DOPPLER DETECTION

(30) Priorité: 27.03.2002 FR 0203833; 09.09.2002 FR 0211152
(43) Date de publication de la demande: 29.12.2004
(73) Titulaire: Bindefeld, Hervé, 75017 Paris (FR)
(72) Inventeur: Bindefeld, Hervé, 75017 Paris (FR)
(74) Mandataire: Schwartz, Thierry J.
(86) Numéro de dépôt international: PCT/FR2003/000972
(87) Numéro de publication internationale: WO 2003/079904

(56) Documents cités:
- US-A- 5 960 089
- US-A- 5 964 709

## Description

L'invention se rapporte à un appareil de dépistage et de diagnostic médical par double détection sonore, de type stéthoscopique, et ultrasonore, de type Doppler. Un tel appareil permet en particulier un diagnostic croisé par couplage de ces détections par analyse auditive et visuelle. L'invention s'applique à l'analyse de la pression artérielle notamment systolique et permet, par exemple, le dépistage d'une sténose vasculaire débutante.

Il est connu actuellement des appareils d'ultrasonographie Doppler comprenant une sonde ultrasonore, équipée de moyens d'émission d'ondes ultrasonores et de réception de ces ondes ultrasonores une fois réfléchies par un support, par exemple le sang s'écoulant dans un vaisseau du patient. Le ralentissement ou l'accélération de cet écoulement se traduit par une variation positive ou négative de la fréquence ultrasonore Doppler, par addition ou soustraction d'un intervalle de fréquences d'amplitude directement liée à l'effet Doppler, ci-après « signal Doppler ». La mesure du signal ou de cette variation peut renseigner fidèlement sur le calibre de la tranche des vaisseaux explorés.

Un appareil d'ultrasonographie Doppler comporte, traditionnellement, outre la sonde ultrasonore, des étages successifs de transducteurs permettant la détection du signal Doppler (déphaseur, multiplieur, amplificateur et filtre), un comparateur de phases attribuant un signe positif ou négatif à l'intervalle Doppler détecté, un amplificateur audiofréquence et un haut-parleur.

US 5 964 709 et US 5 960 089 décrivent des appareils de dépistage et de diagnostic médical par double détection des signaux stéthoscopique et Doppler.

Cependant, l'examen réalisé à l'aide de tels appareils Doppler nécessite une spécialisation, ou tout du moins une formation, afin d'acquérir et de conserver la pratique de l'interprétation des résultats obtenus. Il s'ensuit que, concrètement, cet examen est laissé entre les mains des spécialistes, le médecin généraliste n'ayant pas la culture de ce type de contrôle.

L'invention vise à promouvoir l'examen Doppler afin de permettre des dépistages précoces de maladies cardio-vasculaires, en particulier d'une sténose vasculaire ou d'une artériopathie débutante.

Pour renseigner par exemple sur une artériopathie débutante des membres inférieurs, il convient de déceler un différentiel entre la pression artérielle systolique aux membres inférieurs et aux membres supérieurs du patient. Un tel différentiel est généralement déterminé par la mesure de l'indice de pression systolique (en abrégé IPS, égal au rapport entre les pressions artérielles systoliques au niveau d'un bras et d'un membre inférieur du patient).

Le dépistage facilité d'une artériopathie des membres inférieurs incitera alors à la pratique d'un bilan artériel général, notamment coronarien et carotidien.

Un autre but de l'invention est de réaliser une double détection (stéthoscopique et Doppler) garantissant une haute cohérence entre les détections afin d'obtenir un diagnostic de haute fiabilité.

Pour atteindre ces buts, l'invention utilise des moyens d'intégration de l'examen Doppler dans un appareil de structure typée stéthoscope, faisant partie de la panoplie portative du médecin de ville, ces moyens visant à permettre, spécifiquement, une convergence des explorations stéthoscopique et Doppler d'un même segment vasculaire (réalisant une unité de lieu), avec la possibilité de fournir simultanément (unité de temps) une double détection.

Plus précisément, l'invention a pour objet un appareil de dépistage et de diagnostic médical par double détection des signaux stéthoscopique et Doppler, comportant un conduit de liaison de transmission sonore connecté, sur une extrémité, à un boîtier formant au moins partiellement un cornet acoustique muni d'une membrane et, sur l'autre extrémité, à au moins une oreillette d'écoute d'un signal stéthoscopique provenant du cornet. Dans cet appareil, le boîtier est couplé à au moins une sonde ultrasonore agencée de manière à permettre la convergence en réception des signaux ultrasonores et stéthoscopiques pour fournir simultanément une double détection, et reliée à un circuit de traitement transducteur pouvant fournir, à partir d'un signal Doppler, un signal audio par couplage du circuit de traitement à un haut-parleur en contact avec le cornet pour une écoute de type stéthoscopique, et un signal vidéo par couplage du circuit de traitement à des moyens de visualisation pour fournir une information visuelle.

Ainsi, l'invention permet d'obtenir la convergence simultanée et localisée des explorations stéthoscopique et Doppler sans avoir à déplacer ou à retourner le boîtier pendant les examens. De plus, les résultats sont communiqués à l'aide de moyens d'utilisation traditionnelle ou courante, une oreillette stéthoscopique ou un écran de visualisation, ce qui permet une intégration naturelle de cet appareil dans la panoplie de base du praticien.

Selon des caractéristiques préférées :
- il est prévu des moyens de délivrance et de formation d'un film de produit semi-solide sur la peau du patient, en particulier un gel, pour réaliser un contact intime peau / boîtier et canaliser la propagation des ondes ; un réservoir de ce produit, connecté aux moyens de délivrance, peut être avantageusement logé dans le boîtier ;
- le haut-parleur est disposé sensiblement contre le cornet acoustique pour que le signal audio soit amplifié par le cornet et rende l'écoute stéthoscopique perceptible au niveau de l'oreillette via le conduit de liaison, de la même façon que dans un stéthoscope ;
- un microphone est couplé au cornet afin de recueillir le signal stéthoscopique sonore et le transmettre, sous forme de signal électrique, au circuit de traitement et produire un signal vidéo ;
- les moyens de visualisation se présentent sous la forme d'un écran à cristaux liquides, permettant la visualisation graphique d'un signal stéthoscopique et Doppler, ou sous la forme de diodes électroluminescentes ;
- un microprocesseur est commandé par un algorithme d'interprétation et couplé au circuit de traitement pour permettre une analyse et une combinaison des mesures stéthoscopique et/ou Doppler délivrées par le circuit de traitement, ou bien saisies à partir d'écoutes stéthoscopiques, et pour pouvoir fournir un diagnostic stéthoscopique, Doppler, et/ou croisé ;
- un module d'affichage à trois diodes électroluminescentes monté sur le boîtier visualise l'interprétation, et fournit un diagnostic basé directement sur la mesure du signal Doppler ou un diagnostic croisé basé sur l'algorithme d'interprétation en privilégiant le diagnostic Doppler lorsque les interprétations sont divergentes, chaque diode émettant dans une couleur spécifique correspondant, respectivement, à un diagnostic positif (existence d'une pathologie), un diagnostic négatif (pas de pathologie) ou un diagnostic ininterprétable (trop de doutes dans les mesures) au cas où le signal stéthoscopique est ininterprétable, ou de dysfonctionnement de l'appareil, le diagnostic étant alors basé sur le signal stéthoscopique sonore ;
- un module d'enregistrement et de visualisation du signal vidéo Doppler ou stéthoscopique est prévu par liaison sans fil, par exemple hertzienne ou infrarouge, entre le circuit de traitement électronique et un module de visualisation ou d'impression ;
- des sorties périphériques sont prévues afin de permettre une liaison à un micro-ordinateur, à un casque auditif (auxiliaire) ;
- pour réaliser simplement la mise en service de la sonde, notamment à l'aide d'un doigt, il est prévu un circuit électrique de mise sous tension de la sonde ultrasonore, commandé par un actionneur pouvant être monté sur le conduit de liaison ou sur le boîtier ;
- l'actionneur est un interrupteur multifonctionnel qui sert également de commande sélective aux moyens de délivrance de diagnostics stéthoscopique, Doppler ou croisé par les moyens de visualisation, aux moyens de déclenchement du diagnostic à partir de mesures délivrées par le circuit de traitement ou bien saisies à partir d'écoutes, ainsi qu'au système d'enregistrement et de visualisation à distance ;
- la multifonction est réalisée par des paliers différents, identifiés par une table de décision ou une logique de programmation des connexions des circuits en fonction du nombre de sollicitations de l'interrupteur ;
- une alimentation par pile ou par batterie rechargeable est également prévue.

Selon un premier mode de réalisation, le boîtier forme un cornet acoustique logeant la sonde ultrasonore, en particulier de manière centrée, et des moyens de contact peuvent s'interposer temporairement entre la sonde ultrasonore et la membrane du cornet acoustique, pour transmettre un signal Doppler au circuit de traitement couplé au haut-parleur qui émet le signal audio amplifié dans le cornet acoustique.

Lorsque la sonde ultrasonore est mise hors contact de la membrane, on dispose d'un stéthoscope habituel. Mais lorsque la sonde est mise en contact indirect avec la membrane, on dispose d'un appareil de type Doppler.

Les moyens d'interposition comportent de préférence un ballonnet gonflable coiffant l'extrémité distale de la sonde et un dispositif de gonflage du ballonnet. Ce dispositif de gonflage peut comprendre une tubulure mettant le ballonnet en communication avec une source de liquide, et des moyens, comportant de préférence un bouton, destinés à refouler du liquide de la source dans la tubulure, de manière à gonfler le ballonnet.

L'actionneur de commande et le bouton destiné à refouler du liquide sont de préférence un seul et même bouton pouvant être actionné par un doigt, et il est prévu des moyens destinés à maintenir le refoulement du liquide quand le bouton d'actionnement est relâché, ces moyens pouvant être notamment une bobine électromagnétique appliquant une force magnétique de maintien à un piston, en un matériau magnétique, de refoulement du liquide.

De plus, des moyens de délivrance de produit semi-solide peuvent former un film entre la peau et la membrane.

Selon que l'on gonfle ou non le ballonnet, on dispose d'un appareil Doppler ou d'un stéthoscope traditionnel. On peut ainsi déterminer d'abord approximativement, à l'aide de l'appareil fonctionnant en stéthoscope, l'emplacement anatomique, par exemple carotidien, où il convient de pratiquer une ultrasonographie, puis faire l'opération fine d'ultrasonographie à cet emplacement déterminé d'abord d'une manière approximative mais rapide.

On sait qu'il est souhaitable, dans un appareil Doppler, de pouvoir faire basculer la sonde. Dans ce premier mode de réalisation, il est avantageusement prévu des moyens commandés de l'extérieur du cornet et destinés à faire basculer la sonde. Ces moyens peuvent comporter un câble, de préférence un ensemble de quatre câbles aux quatre points cardinaux de la sonde, dont une extrémité est fixée à l'extrémité de la sonde, et des moyens destinés à tirer l'autre extrémité du câble. Le câble, ou une partie de l'ensemble des câbles, est tiré(e) de manière à faire basculer l'extrémité de la sonde pour l'orienter vers la réponse sonore la plus perceptible à l'oreillette.

Selon un deuxième mode de réalisation, la sonde est logée dans le boîtier et hors du cornet, le boîtier formant une tourelle sensiblement cylindrique.

La sonde peut être inclinée d'un angle fixe entre environ 30 et 70 degrés par rapport à la membrane, de préférence entre 40 et 55 degrés, afin d'optimiser l'examen en faisant converger la réception des signaux ultrasonores et celle des signaux stéthoscopiques.

Avantageusement, des moyens de prolongement de la sonde sont prévus afin de compenser l'inclinaison de la sonde. Les moyens de délivrance du produit semi-solide forment alors une couche de liaison entre le prolongement de la sonde et la peau du patient.

Selon une caractéristique préférée, le conduit de liaison est connecté au fond du cornet, au plus loin de la membrane, et émerge sensiblement au centre de la face supérieure de la tourelle.

D'autres avantages et caractéristiques de l'invention apparaîtront à la lecture de la description qui suit, relative à des exemples de réalisation non limitatifs et accompagnée de figures annexées qui représentent :
- la figure 1, une vue d'ensemble, partiellement en coupe, d'un exemple d'appareil selon l'invention, dans un premier mode de réalisation ;
- la figure 2, une vue en coupe d'une partie du conduit de liaison de l'appareil de la figure 1, dans laquelle un bouton actionneur et les circuits de commande apparaissent schématiquement ;
- la figure 3, une vue en coupe de l'embout de cet appareil en position de fonctionnement stéthoscopique ;
- les figures 4 et 5, une vue en coupe selon la figure 3 en position de fonctionnement Doppler dans lesquelles le ballonnet de la sonde est gonflé et la sonde émet et reçoit des ondes ultrasonores avec, respectivement, enclenchement et relâchement du bouton actionneur ;
- les figures 6 à 9, une vue semblable à celle des figures 4 et 5, dans lesquelles le bouton d'actionnement bascule, pour optimiser la réponse du signal Doppler, puis est relâché, puis est actionné de nouveau pour mettre l'ensemble des circuits de traitement de l'appareil hors tension, et enfin relâché pour revenir en position de fonctionnement stéthoscopique ;
- les figures 10a et 10b, deux vues en perspective supérieure et inférieure d'un boîtier d'appareil selon l'invention, dans un deuxième mode de réalisation ;
- la figure 11, une vue en coupe du boîtier selon les figures 10a et 10b dans un exemple de réalisation adapté au traitement prioritaire du signal Doppler et du signal stéthoscopique en mode audio ;
- la figure 12, un exemple de circuit de traitement des signaux Doppler de l'appareil selon l'invention ;
- la figure 13, une vue en coupe du boîtier dans un exemple de réalisation adapté au traitement prioritaire du signal Doppler et du signal stéthoscopique en mode vidéo ;
- la figure 14, une vue en coupe du boîtier dans une variante de réalisation de l'exemple précédent; et
- les figures 15 et 16, deux autres variantes de validation clinique d'une vue en coupe d'exemples d'appareil selon l'invention présentant une structure de type sthétoscope.

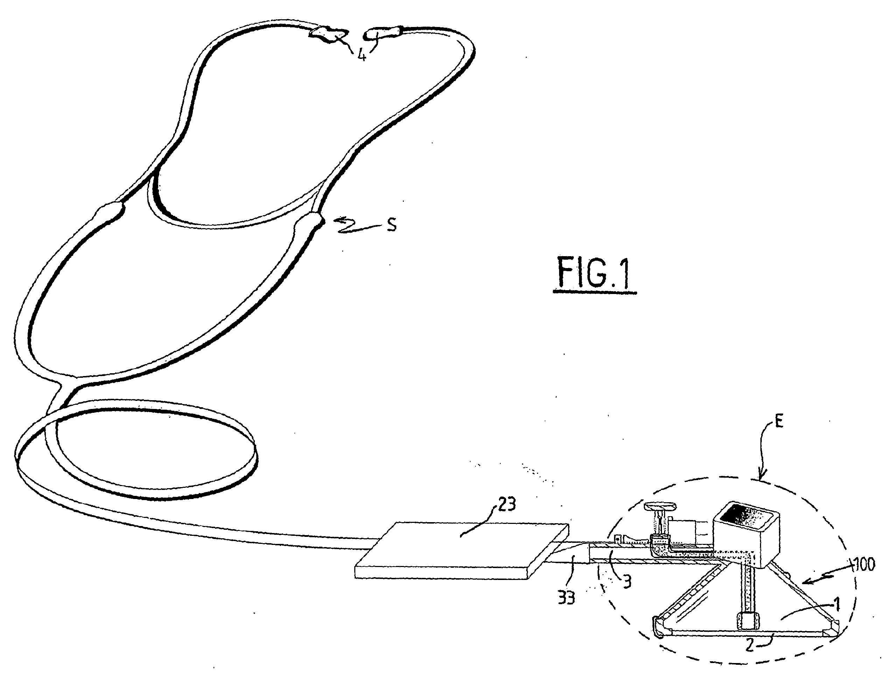

L'appareil représenté à la figure 1 comprend un embout de stéthoscope E dont le boîtier 100 est en forme de cornet conique 1 délimité également par une membrane 2 formant la grande base du cornet, et comportant un conduit de liaison constitué d'un raccord 3 et d'un tuyau 33 flexible communiquant avec deux oreillettes 4 à la manière, classique, d'un stéthoscope S, et sur lequel est fixée une source électrique 23.

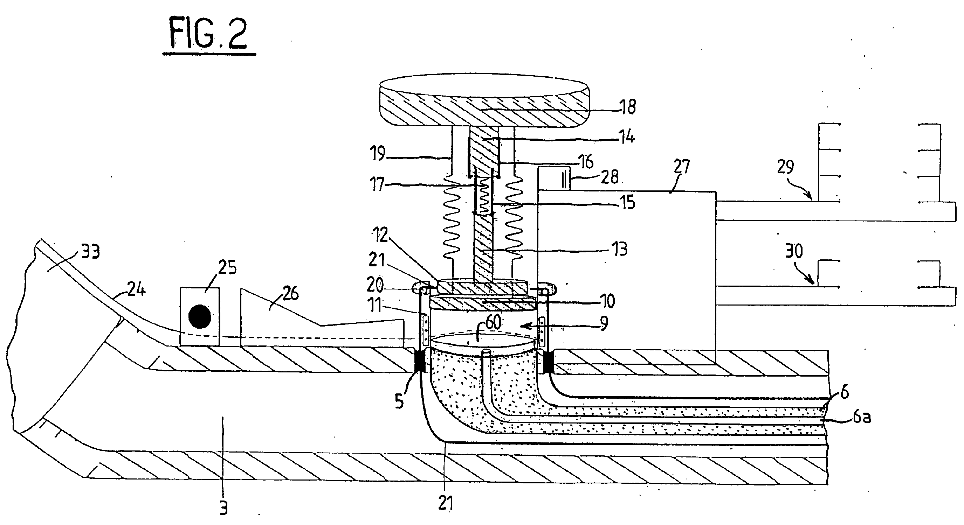

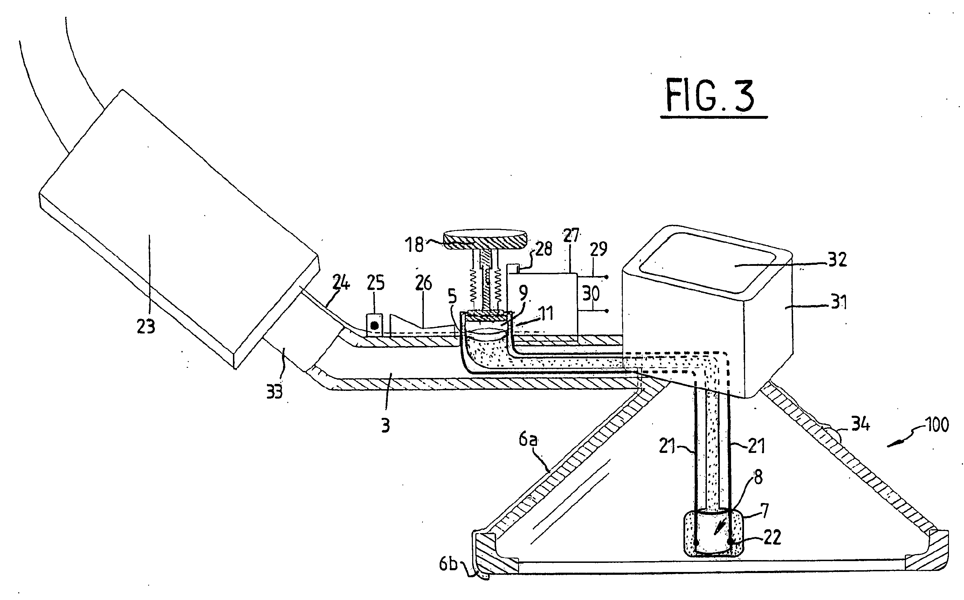

Mais à la différence d'un stéthoscope habituel, il est prévu comme illustré plus précisément à la figure 2 et par la vue globale de l'embout en figure 3, sur le raccord 3 un trou 5 avec garniture d'étanchéité, dans lequel passe une tubulure 6, débouchant dans un ballonnet 7 coiffant l'extrémité distale d'une sonde ultrasonore 8. La tubulure 6 est emplie de liquide, par exemple de l'eau.

Dans le trou 5 est engagé un cylindre 9 dans lequel coulisse un piston 10 en matériau magnétique, et qui est entouré d'une bobine électromagnétique 11. Le piston 10 est coiffé d'un disque 12 dont la face supérieure est solidaire d'une tige 13. La tige 13 est montée de manière télescopique sur la tige 14 d'un bouton actionneur 18 par l'intermédiaire de deux rails 15, pouvant coulisser sur deux autres rails 16, eux-mêmes solidaires de la tige 14, un ressort 17 étant interposé entre les faces en regard des tiges 13 et 14. Le bouton actionneur 18 est relié à la face supérieure du piston 10 par un ressort 19.

Un réservoir de gel 60 est disposé dans le cylindre 9, entre le piston 10 et la tubulure 6. Ce réservoir est relié à un tube souple 6a monté dans la tubulure 6 puis, émergeant par des orifices étanches de celle-ci et du raccord 3, le long de la paroi conique du cornet 1. L'extrémité du tube 6a est reliée à une buse d'éjection 6b située au contact de la face externe de la membrane 2. Alternativement, le système de distribution de gel et sa commande peuvent être montés indépendamment du système de gonflage du ballonnet et de sa commande.

Au cylindre 9 sont fixées quatre poulies 20 sur lesquelles passent quatre câbles 21 dont les extrémités sont fixées en 22 à la sonde 8. Les autres extrémités des câbles 21 sont fixées au disque 12.

Sur le tuyau 33 est montée une pile ou une batterie rechargeable 23 connectée, par une ligne 24, à une prise 25 de recharge et à un interrupteur 26.

Le bouton 18 commande également un module de traitement électronique 27 par un bouton poussoir 28 se trouvant sous le bouton de commande 18, de sorte que ce dernier entraîne le bouton poussoir 28 lorsqu'il est sollicité. Le module de traitement électronique comporte les circuits transducteurs de conversion des signaux Doppler en signaux sonores et vidéo.

Le module de traitement électronique 27 est connecté à des premier et second circuits, 29 et 30, de liaison électrique notamment à un microprocesseur 31 et à un dispositif de visualisation à cristaux liquides 32. Le premier circuit 29 électrique alimente la bobine 11 électromagnétique, ainsi que le microprocesseur et l'écran. Il met sous tension la sonde 8 et un haut-parleur 34 disposé contre une paroi du cornet conique 1. Le second circuit électrique 30 est un circuit qui commande l'enregistrement du signal Doppler par le microprocesseur 31 et son affichage sur le dispositif 32.

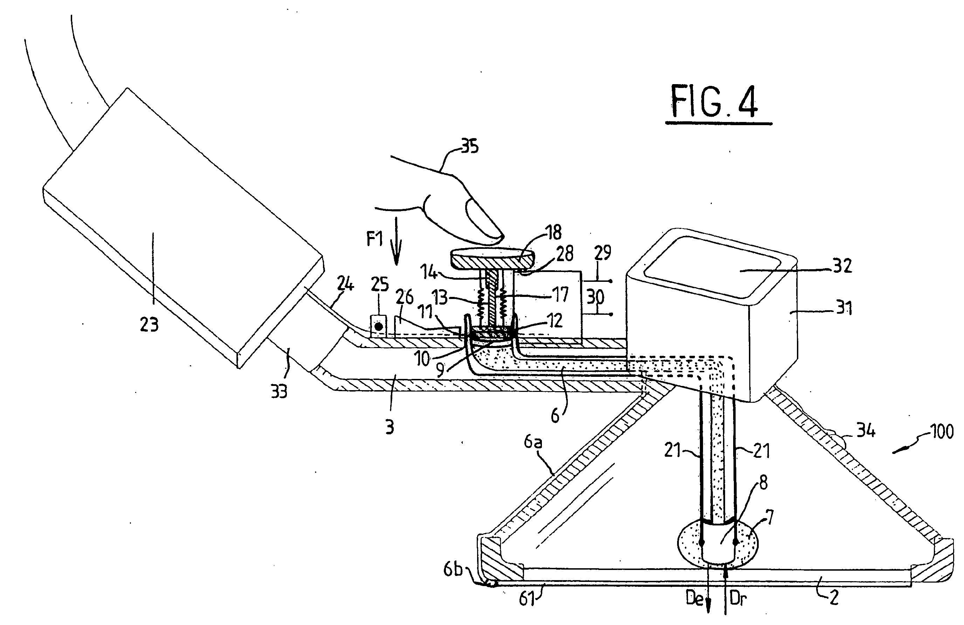

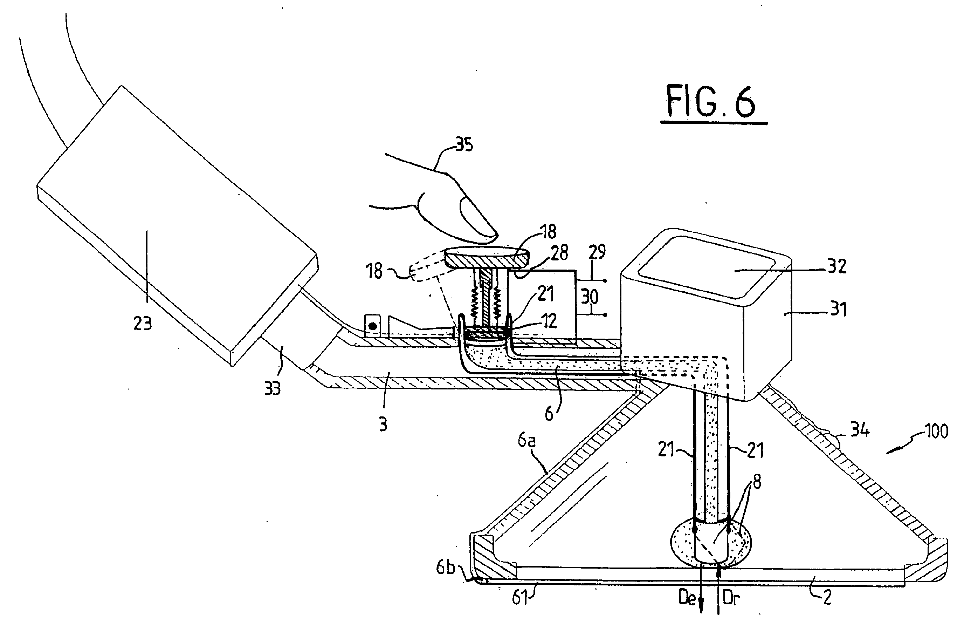

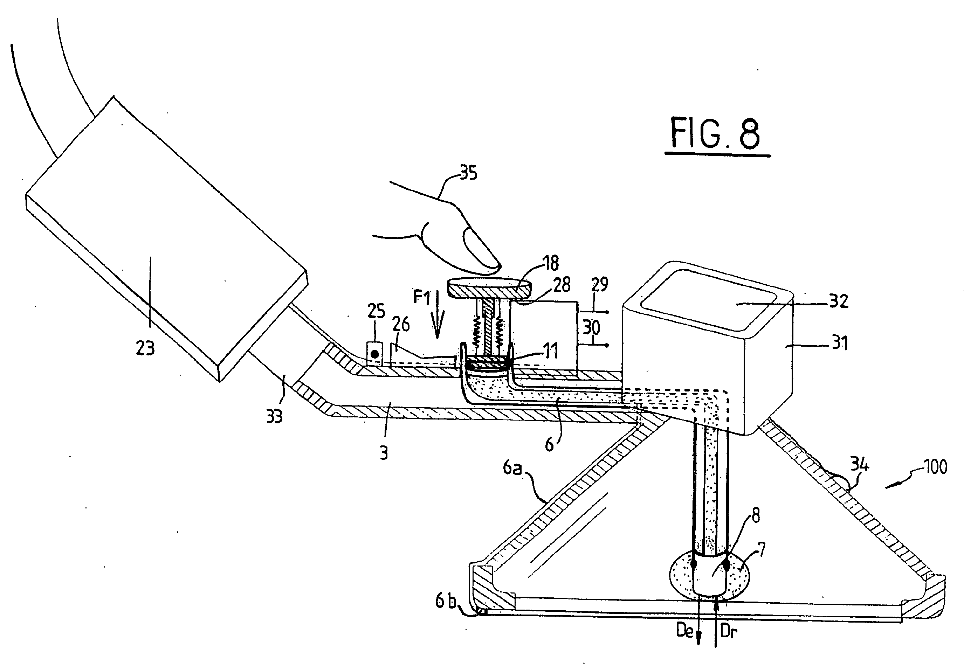

Le module 27 a une table de décision telle qu'une première sollicitation du bouton 28, par appui de l'actionneur 18, nous installe dans la configuration illustrée par la figure 4. Après avoir relâché, une nouvelle sollicitation nous transporte dans la configuration selon la figure 6. Un nouveau relâchement suivi d'une nouvelle sollicitation nous amène en la position de la figure 8.

L'appareil fonctionne de la manière suivante.

En référence à la figure 3, dans laquelle le ballonnet 7 n'est pas gonflé et la sonde 8 n'est pas mise sous tension (pouce 35 non actif), l'appareil fonctionne comme un stéthoscope en fournissant un signal audio stéthoscopique habituel par propagation du son perceptible à travers la membrane 2, le cornet 1 et le tuyau flexible 33 aux oreillettes 4.

En figure 4, le bouton 18 est actionné par le pouce 35 (flèche F1). Le doigt 14 repousse la tige 13 qui repousse elle-même le disque 12 vers le bas, lequel fait coulisser le piston 10 dans le cylindre 9. Le ressort 17 est comprimé.

Le liquide contenu dans la tubulure 6 est refoulé dans le ballonnet 7, qui vient en contact avec la membrane 2. Le gel 61 est diffusé par la buse 6b entre la membrane 2 et la peau du patient.

Dans le même temps, le bouton 18 a appuyé une première fois sur le bouton 28, en sorte que le premier circuit 29 électrique est mis sous tension. La bobine 11 électromagnétique est mise sous tension et maintient le piston 10 en matériau magnétique en place, en sorte que le liquide contenu dans la tubulure 6 reste refoulé et que le ballonnet 7 reste gonflé. Le microprocesseur 31 et l'écran 32 sont mis sous tension.

A partir d'éléments piézoélectriques, la sonde 8 également mise sous tension émet des ultrasons De à travers la membrane 2 et reçoit, en retour, les ultrasons réfléchis Dᵣ complétés d'un intervalle de fréquence Doppler pour former le signal Doppler. L'appareil fonctionne alors en tant qu'appareil d'ultrasonographie Doppler. Le signal Doppler est converti par le module de traitement 27 pour fournir un signal sonore par l'intermédiaire du haut-parleur 34. Le son est ensuite amplifié dans le cornet, propagé dans le conduit de liaison (raccord 3 et tuyau 33), puis écouté au niveau des oreillettes 4. Il est ainsi possible d'effectuer un examen Doppler par écoute stéthoscopique.

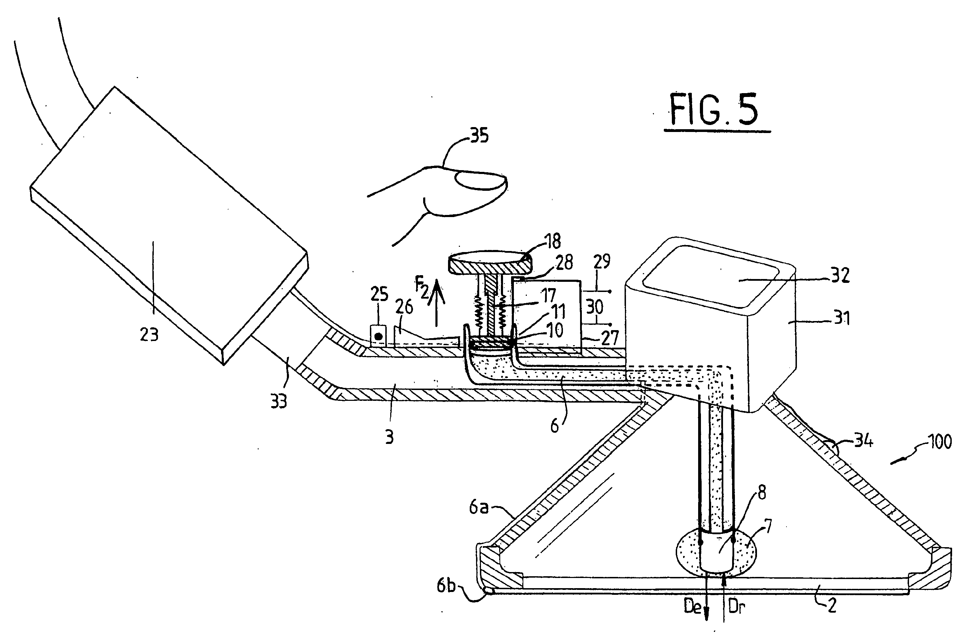

En figure 5, on a cessé d'appuyer sur le bouton 18 (flèche F2), mais le piston 10 est resté en position du fait que la bobine 11 est mise sous tension, le bouton 28 étant rappelé vers le haut par le ressort 17.

En figure 6, on a représenté en trait mixte un basculement du bouton 18 par le pouce 35 pour optimiser la réception du signal Doppler. Ce basculement du bouton 18 se traduit également par un basculement du disque 12. Il s'ensuit que certains des câbles 21 sont tirés par le disque 12, et font basculer l'extrémité de la sonde 8. En écoutant par l'oreillette 4, on entend un son qui, en une certaine orientation du bouton 18 et donc de la sonde 8, atteint un maximum. Lorsque cette orientation est atteinte, on appuie à nouveau sur le bouton 18, tout en lui conservant son orientation. Il appuie sur le bouton 28, ce qui met sous tension le deuxième circuit 30. On enregistre le signal Doppler par le microprocesseur 31 et on lit le résultat sur l'écran d'affichage 32.

Le microprocesseur peut être commandé par un logiciel d'interprétation des résultats Doppler et stéthoscopiques. Les résultats stéthoscopiques sont saisis par des touches dédiées ou par l'écran tactile. Lorsque les résultats diffèrent, le résultat Doppler est prépondérant dans l'interprétation. Le diagnostic final est affiché sous forme d'un résultat positif (pathologie), négatif (pas d'anomalie) ou de résultat ininterprétable lorsque les signaux ne sont pas suffisamment identifiés ou sûrs. Dans le cas où seul le signal Doppler n'est pas exploitable, le diagnostic est celui - positif ou négatif - fourni par le signal stéthoscopique.

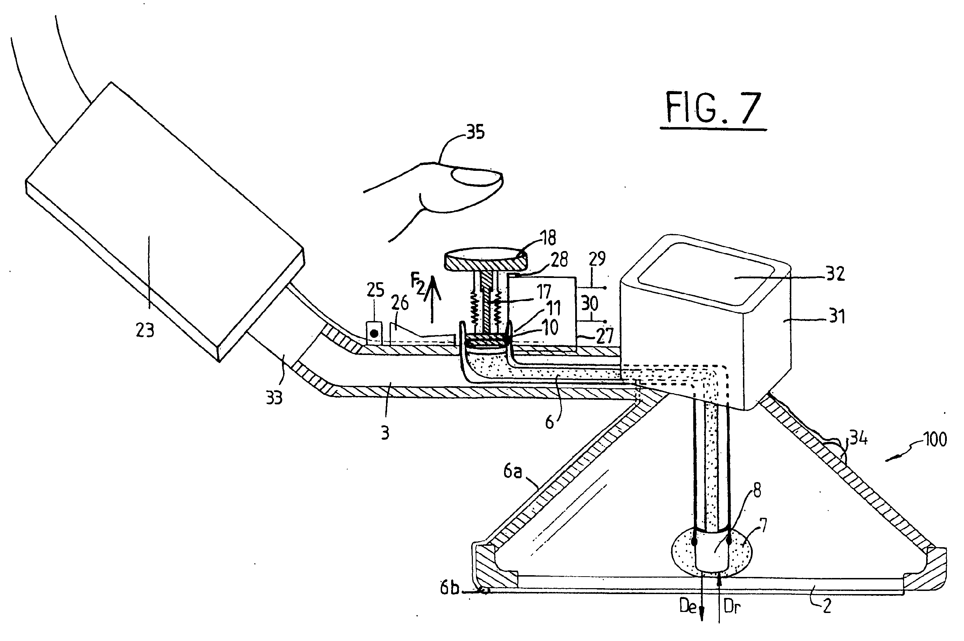

En figure 7, on a cessé d'appuyer sur le bouton 18 (flèche F2). Le piston 10 reste maintenu par la bobine 11 électromagnétique, de sorte que le ballonnet 7 reste gonflé. L'affichage de l'enregistrement sur le dispositif 32 se poursuit. En figure 8, on appuie à nouveau sur le bouton 18 (flèche F1), ce qui appuie sur le bouton 28. On coupe ainsi les premier et second circuits 29 et 30, et on met ainsi hors tension la bobine 11.

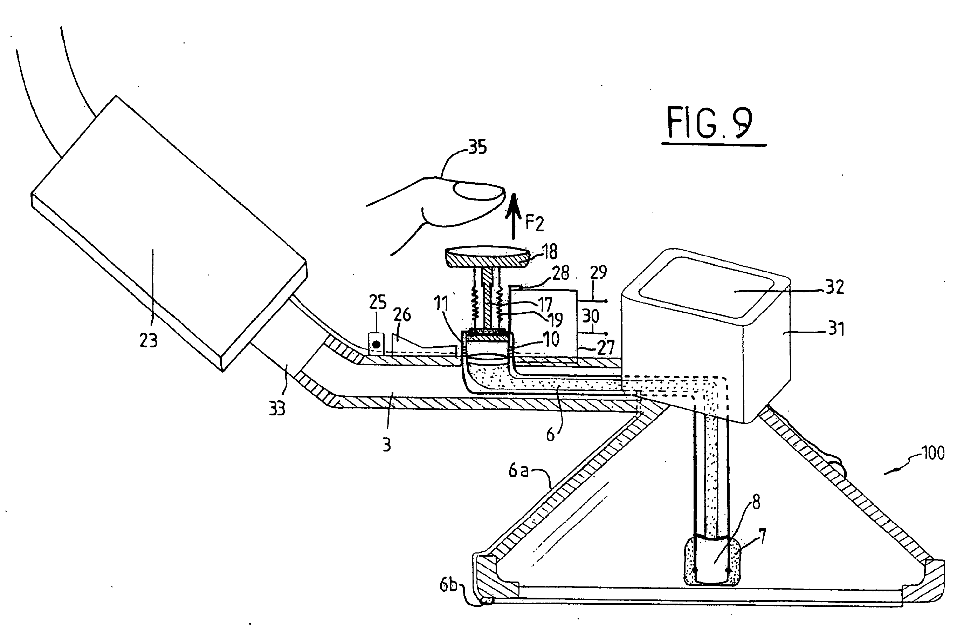

En figure 9, on a cessé d'appuyer sur le bouton 18. Le piston 10, qui n'est plus retenu par la bobine 11 remonte, sous l'effet des ressorts 17 et 19. Le liquide contenu dans la tubulure 6 est aspiré. Le ballonnet 7 se dégonfle. La sonde 8 a cessé d'émettre. Si on le souhaite, on peut appuyer sur l'interrupteur 26 de mise hors circuit de tout l'appareil pour ménager la pile 23.

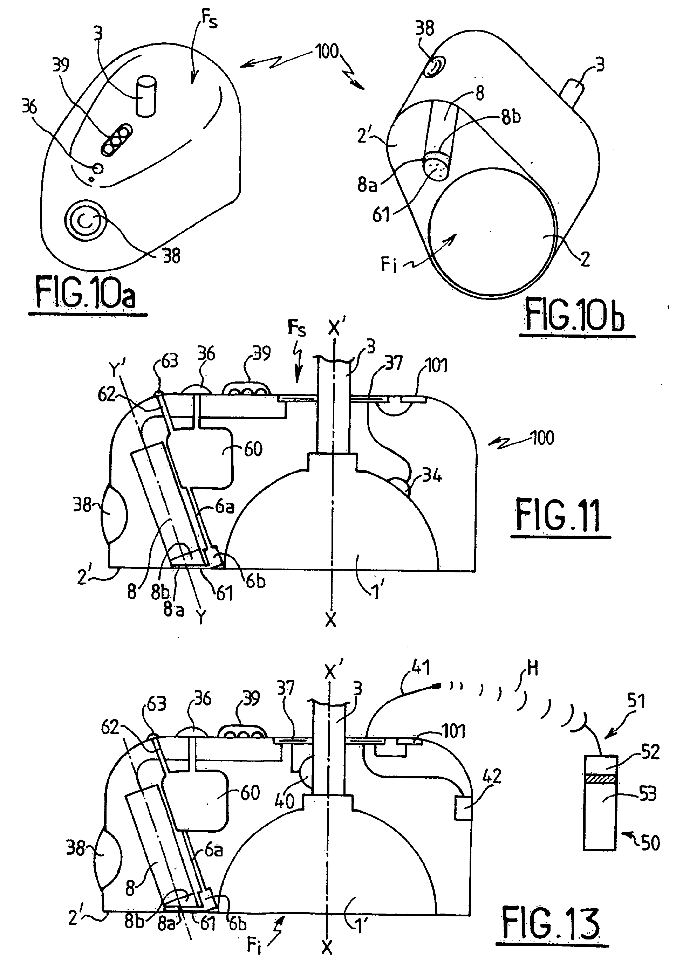

Les figures 10a et 10b illustrent les vues d'un boîtier d'appareil selon l'invention dans un deuxième mode de réalisation. Dans ce deuxième mode, la sonde 8 est logée dans le boîtier 100, hors du cornet 1' en forme de coupole.

Le boîtier 100 présente une forme de tourelle, sensiblement cylindrique et de section ovoïde. La tourelle est limitée par une face supérieure de sortie Fs, au centre de laquelle émerge le raccord 3, et par une face inférieure ouverte Fi d'application, où viennent se placer la membrane plane 2 du cornet et la face d'extrémité 8a d'un prolongement 8b de la sonde inclinée 8. Ce prolongement est en matériau solide ou semi-solide conducteur des ondes ultrasonores, par exemple en gel de silicone. Dans l'exemple illustré, il présente une forme cylindrique à faces d'extrémité planes et sectionnées selon des angles adaptés.

La face d'extrémité 8a de la sonde prolongée est recouverte d'un gel 61 pour former une couche de liaison continue entre l'extrémité 8a et la peau du patient. Une membrane isolante 2', percée de façon à laisser l'extrémité 8a libre, peut avantageusement fermer la face inférieure de la tourelle afin d'éviter la pénétration du gel à l'intérieur de la tourelle.

Le gel est commandé par un piston 36 accessible de la face supérieure Fs. Un interrupteur 38 de mise en tension de la sonde 8 et un module d'affichage 39 sont également disposés sur le boîtier, le module d'affichage étant sur la face supérieure Fs dans l'exemple illustré. L'interrupteur 38 sert également de commande multifonctionnelle comme décrit en référence au premier mode de réalisation.

La vue en coupe illustrée en figure 11 présente une version adaptée au traitement prioritaire des signaux en mode audio.

L'axe Y'Y de la sonde est inclinée par rapport à l'axe central X'X de symétrie du cornet, d'un angle fixe d'environ 50 degrés par rapport au plan de la membrane 2'. Ce type d'inclinaison permet de réaliser la double détection par une convergence des signaux Doppler et de l'écoute stéthoscopique en aval de l'appareil, plus précisément sur le site d'exploration.

Dans cet exemple de réalisation, comme dans les suivants, le gel 61 provient d'un réservoir 60 disposé dans le boîtier. Le gel est délivré à travers un tube souple 6a par une buse d'éjection 6b, située en contact de la face inférieure Fi de la tourelle. Dans le cas où une membrane isolante 2' est utilisée, la buse 6b la traverse par un orifice autour duquel une garniture d'étanchéité est prévue.

La poussée du piston 36, monté de manière télescopique comme précédemment ou simplement sur ressort de rappel, permet de doser la quantité adéquate de gel délivrée par la buse. Le réservoir peut être rechargé à travers un tuyau souple 62 qui relie le réservoir 60 à une embouchure de réassort 63 intégrée au boîtier.

Toujours dans cet exemple, la sonde 8 est reliée à un haut-parleur 34, monté sur une face externe du cornet 1' via le circuit transducteur 37. Le signal Doppler est converti par le circuit transducteur 37 pour fournir un signal audio par le haut-parleur 34. Comme précédemment, le son est amplifié dans le cornet, propagé dans le conduit de liaison, 3 et 33, puis écouté au niveau des oreillettes.

Les résultats des écoutes stéthoscopique et Doppler peuvent être saisis et mémorisés, après évaluation par l'opérateur, dans un logiciel d'interprétation. Ce logiciel commande le microprocesseur d'un micro-ordinateur (non représenté) branché sur une sortie 101 prévue sur le boîtier 100. Alternativement ou cumulativement, le signal Doppler, après conversion par le circuit 37, est également transmis au micro-ordinateur et mémorisé sous forme de signal vidéo à travers la sortie 101. Le micro-ordinateur est équipé d'un écran qui visualise le graphe du signal Doppler.

Le logiciel a pour base un algorithme qui fournit un diagnostic à partir des évaluations des écoutes et du signal Doppler vidéo. Le module d'affichage 39 est équipé de trois diodes électroluminescentes. Il est monté sur la face supérieure Fs du boîtier 100 et couplé au circuit transducteur 37. Ce module permet de visualiser l'interprétation. L'algorithme d'interprétation privilégie le diagnostic Doppler lorsque les interprétations sont divergentes.

Les diodes émettent respectivement en lumière rouge, orange et verte :
- l'émission de la diode rouge signifie que le diagnostic est positif (existence d'une pathologie),
- l'émission de la diode verte signifie que le diagnostic est négatif (pas de pathologie), et
- l'émission de la diode orange signifie que le résultat est ininterprétable, du fait l'existence de mesures trop « limites ».

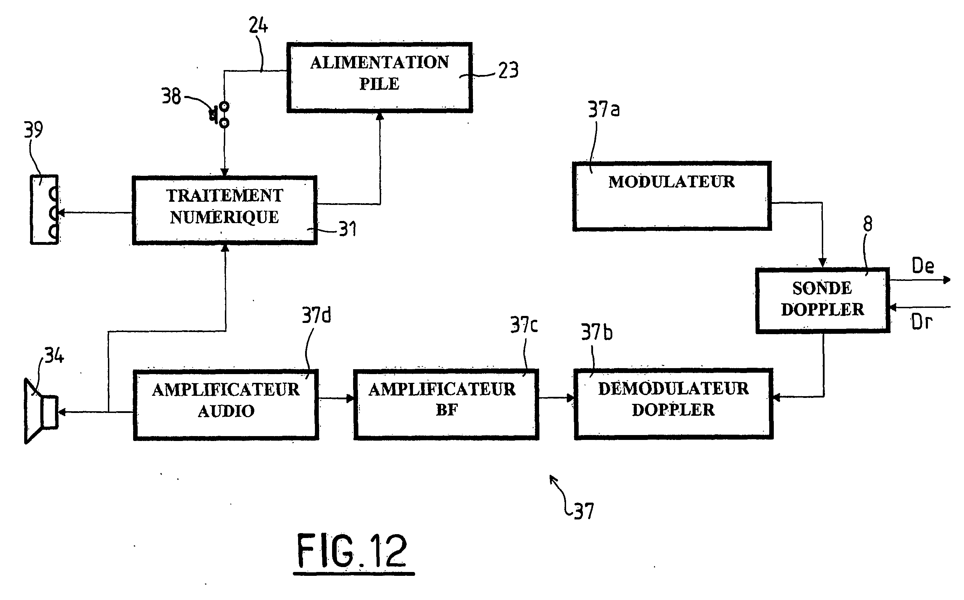

Un exemple de circuit transpondeur 37 de traitement des signaux Doppler est illustré en figure 12. Ce circuit comporte un modulateur 37a d'impulsion du signal émis De à 4 MHz, couplé à la sonde 8. Le traitement du signal reçu Dᵣ par la sonde pour être transmis au haut-parleur 34 est effectué par les composants en série suivants :
- un démodulateur basses fréquences 37b par exemple inférieures à 8 kHz ;
- un amplificateur 37c, pour atteindre quelques dizaines à quelques centaines de mV ; et
- un amplificateur audio 37d, en tension et impédance.

Le signal est transmis également au microprocesseur de traitement numérique 31. Ce microprocesseur commande le circuit d'alimentation 24 par pile 23 à travers l'interrupteur 38, ainsi que la visualisation du diagnostic par le module d'affichage à diodes 39 précédemment décrit.

Le déclenchement des diodes se fait par comptage du nombre de fronts d'impulsions reçues par un « trigger » de Schmidt (bascule électronique), qui permet de s'affranchir des impulsions parasites. Par exemple, en nombre de fronts par seconde :
- moins de 500 fronts : diode orange (nombre insuffisant : résultat ininterprétable) ;
- entre 500 et 2000 fronts : diode verte déclenchée ;
- entre 4000 et 8000 fronts : diode rouge déclenchée
- plus de 8000 fronts : diode orange (nombre trop élevé : résultat ininterprétable).

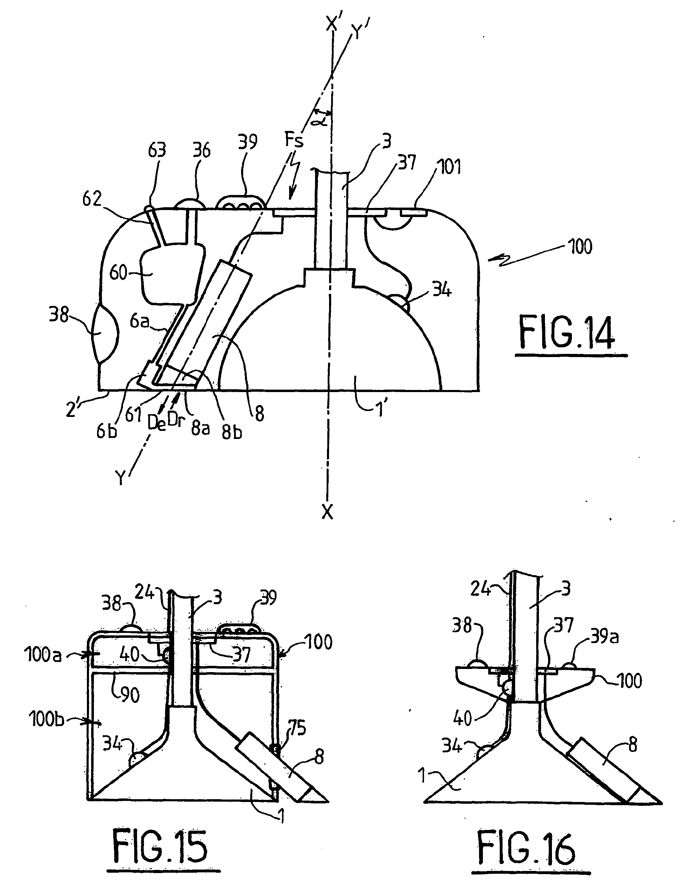

La figure 13 illustre plus particulièrement une autre version du deuxième mode de réalisation, adaptée au traitement prioritaire des signaux en mode vidéo. Dans cet exemple, un microphone 40 est apposé contre la paroi externe du raccord 3. Alternativement, il peut être agencé dans le conduit de liaison, dans la mesure où cette position ne provoque pas d'atténuation de l'intensité du signal sonore direct, ou contre la paroi du cornet acoustique 1', ou plus généralement sur tout emplacement approprié situé sur le trajet sonore formé par le cornet.

Ce microphone permet de recueillir le signal stéthoscopique sonore et est relié au circuit de transduction 37 afin de le transmettre, sous forme de signal électrique, et produire en sortie un signal vidéo. De plus, le signal Doppler reçu par la sonde 8 est également converti en signal vidéo.

Les signaux vidéo sont transmis à un micro-ordinateur, comme dans l'exemple précédent (illustré en figure 11) par la sortie 101, et/ou vers un module de visualisation et d'impression 50, situé à distance. A cette fin, une antenne 41 est prévue pour émettre en ondes hertziennes les signaux vidéo.

Un signal H est alors capté par le récepteur 51 du module de visualisation, puis traité dans un démodulateur 52 et dans un adaptateur de visualisation 53.

Par ailleurs, une sortie casque 42 est également prévue dans cet exemple afin de permettre une écoute stéthoscopique à partir du son capté par le microphone, ou à partir du signal Doppler converti en signal audio par le circuit 37. Le casque auditif peut se présenter sous une forme conventionnelle à oreillettes 4 typée stéthoscope.

Comme dans l'exemple précédent, les signaux vidéo (ou audio après saisie manuelle de l'interprétation de l'opérateur) sont évalués dans le logiciel d'interprétation du microprocesseur ou visualisés par l'écran du micro-ordinateur.

Le module d'affichage à trois diodes électroluminescentes 39 permet une interprétation croisée à partir des signaux vidéo Doppler et stéthoscopique, le signal Doppler étant prépondérant en cas de divergence.

La vue en coupe de la figure 14, qui illustre une variante de réalisation du boîtier des figures 11 et 13, montre la sonde 8 selon une position inclinée par rapport à l'axe central X'X, avec une inclinaison opposée à celle précédemment décrite et représentée. La convergence de l'axe Y'Y de la sonde 8 et de l'axe central X'X ne se produit plus en aval du boîtier, comme dans l'exemple précédent, mais en amont du boîtier 100, au niveau du tuyau flexible 33. Cette solution permet de réaliser une double détection qui privilégie l'identité de la portion de courant sanguin, examinée successivement en fonction de son écoulement, par rapport à l'identité du site d'exploration.

Avantageusement, un micro-moteur électrique 70, commande l'inclinaison a d'un étui 71 dans lequel la sonde 8 est glissée. L'étui et la sonde sont alors entraînés en rotation autour d'un axe perpendiculaire à l'axe central X'X de manière à ajuster l'angle d'inclinaison α en fonction du signal de réception de manière à optimiser l'exploration Doppler.

Les figures 15 et 16 illustrent deux autres variantes de validation clinique de l'appareil selon l'invention, présentant une structure simplifiée de type stéthoscope.

En référence à la figure 15, une cloison 90 sépare le boîtier 100 en une partie supérieure 100a de traitement de signal, comportant le circuit transpondeur 37, le système de diodes 39 et l'interrupteur 38, et une partie inférieure 100 b d'acquisition de signal, dans laquelle le microphone 40, le haut-parleur 34, le cornet 1, le circuit d'alimentation 24 et la sonde 8 assurent les mêmes fonctions que précédemment.

Le boîtier 100 loge partiellement la sonde 8 et une bague d'étanchéité 75 est positionnée dans la découpe du boîtier 100, autour de la sonde 8, afin d'enchâsser et isoler mécaniquement la sonde. En effet, la bague permet la fixation de la sonde au boîtier sans perturber les vibrations normales des éléments piézo-électriques de la sonde. On obtient ainsi un signal Doppler de réception optimal.

En variante de cet exemple, la partie inférieure 100b du boîtier peut être avantageusement cintrée dans sa partie centrale pour favoriser une prise en main du boîtier de type stéthoscopique.

L'exemple de la figure 16 illustre une solution encore plus proche du stéthoscope traditionnel. Dans cette variante, le boîtier 100 ne loge plus la sonde 8 et se réduit à la partie supérieure 100a de traitement de signal (figure 15). La sonde 8 est fixée le long du cornet 1 par des moyens adaptés, connus de l'homme de l'art. Le système à diodes est réduit à une seule diode 39a, apte à prendre sucessivement les trois couleurs précédemment décrites. Cette diode peut également remplacer le système à trois diodes décrit ci-dessus dans les exemples de réalisation précédents.

L'invention n'est pas limitée aux exemples de réalisation décrits et représentés. Il est par exemple possible de prévoir, dans le deuxième mode de réalisation, le microprocesseur et le module de visualisation tels qu'agencés dans le premier mode de réalisation, ou de prévoir le module de visualisation à diodes dans tout exemple décrit ci-dessus.

Une deuxième sonde peut également être logée dans le boîtier. Chacune des deux sondes est alors dédiée à une zone particulière du corps, par exemple partie supérieure (membres supérieurs ou cou) et partie inférieure (membres inférieurs) du patient. Les signaux respectifs des sondes sont réglés sur une fréquence dédiée, par exemple 4 et 8 MHz. D'autres sondes peuvent également être prévues, dont les extrémités sont par exemple inscrites dans une couronne périphérique, intérieure ou extérieure au cornet acoustique. Il est alors possible de prévoir une enveloppe annulaire pour former une sonde annulaire unique à émetteurs et récepteurs piézoélectriques multiples.

De plus, une sortie casque peut également être prévue afin de brancher un câble de casque à la place ou en plus du conduit de liaison stéthoscopique, dans tout exemple. Un système d'enregistrement et de visualisation du signal vidéo Doppler ou stéthoscopique peut être est prévu dans tout mode de réalisation, par liaison sans fil, par exemple hertzienne ou infrarouge, entre le circuit de traitement électronique et un module de visualisation ou d'impression.

Le piston de commande du produit semi-solide peut être accessible à partir du boîtier, l'interrupteur de mise en tension de la sonde étant également disposé en tout emplacement approprié situé sur le boîtier.

Par ailleurs, il est possible de transmettre les trois types d'information de diagnostic par synthèse vocale à la place d'une visualisation par diodes. La voix synthétique est obtenue à l'aide d'un synthétiseur couplé au microprocesseur et transmise par le conduit de liaison aux oreillettes d'écoute stéthoscopique.

Il est également possible de prévoir un système de contrôle (oscillométrique ou autre) de la charge de la pile, couplé à des moyens d'alerte indiquant la nécessité de recharger ou de remplacer la batterie ou la pile lorsque le niveau de charge est inférieur à un certain seuil. Un tel système peut comprendre des moyens d'interruption du système de visualisation ou des moyens d'interprétation, afin de ne pas fournir de résultats erronés lorsque le niveau de charge atteint ledit seuil.

## Revendications

1. Appareil de dépistage et de diagnostic médical par double détection des signaux stéthoscopique et Doppler, comportant un conduit de liaison (3, 33) de transmission sonore connecté, sur une extrémité, à un boîtier (100) formant au moins partiellement un cornet acoustique (1,1') muni d'une membrane (2) et, sur l'autre extrémité, à au moins une oreillette d'écoute (4) d'un signal stéthoscopique provenant du cornet, **caractérisé en ce que** le boîtier (100) est couplé à au moins une sonde ultrasonore (8), agencée de manière à permettre la convergence en réception des signaux ultrasonores et stéthoscopiques pour fournir simultanément une double détection, et reliée à un circuit de traitement transducteur (37) pouvant fournir, à partir d'un signal Doppler, un signal audio par couplage du circuit de traitement (37) à un haut-parleur (34) en contact avec le cornet (1, 1') pour une écoute de type stéthoscopique, et un signal vidéo par couplage du circuit de traitement (37) à des moyens de visualisation (31, 32, 39) pour fournir une information visuelle.

2. Appareil de dépistage et de diagnostic médical selon la revendication précédente, dans lequel il est prévu des moyens de délivrance (60, 6a) et de formation (6b) d'un film de produit semi-solide (61) sur la peau du patient, en particulier un gel, pour réaliser un contact intime peau / boîtier et canaliser la propagation des ondes.

3. Appareil de dépistage et de diagnostic médical selon la revendication 1 ou 2, dans lequel il est prévu un haut-parleur (34) disposé sensiblement contre le cornet acoustique (1, 1') pour que le signal audio soit amplifié par le cornet et rende l'écoute stéthoscopique perceptible au niveau de l'oreillette (4) via le conduit de liaison (3, 33), de la même façon que dans un stéthoscope.

4. Appareil de dépistage et de diagnostic médical selon l'une quelconque des revendications 1 à 3, dans lequel il est prévu un microphone (40) couplé au cornet (1,1') afin de recueillir le signal stéthoscopique sonore et le transmettre, sous forme de signal électrique, au circuit de traitement (37) et produire un signal vidéo.

5. Appareil de dépistage et de diagnostic médical selon l'une quelconque des revendications précédentes, dans lequel les moyens de visualisation se présentent sous la forme d'un écran à cristaux liquides (32), permettant la visualisation graphique d'un signal stéthoscopique et Doppler, ou sous la forme d'un module à diodes électroluminescentes (39).

6. Appareil de dépistage et de diagnostic médical selon l'une quelconque des revendications précédentes, **caractérisé en ce qu'**il comprend un microprocesseur commandé par un algorithme d'interprétation et couplé au circuit de traitement (37) pour permettre une analyse et une combinaison des mesures stéthoscopique et/ou Doppler, délivrées par le circuit de traitement (37) ou bien saisies à partir d'écoutes stéthoscopiques, et pour pouvoir fournir un diagnostic stéthoscopique, Doppler, et/ou croisé.

7. Appareil de dépistage et de diagnostic médical selon l'une quelconque des revendications précédentes, **caractérisé en ce qu'**il comprend un module d'affichage à trois diodes électroluminescentes (39) monté sur le boîtier (100), qui visualise l'interprétation et fournit un diagnostic basé sur la mesure du signal Doppler ou un diagnostic croisé basé sur l'algorithme d'interprétation en privilégiant le diagnostic Doppler lorsque les interprétations sont divergentes, chaque diode du module (39) émettant dans une couleur spécifique correspondant, respectivement, à un diagnostic positif, un diagnostic négatif ou un résultat ininterprétable dans le cas où au moins la mesure Doppler est ininterprétable.

8. Appareil de diagnostic médical selon la revendication précédente, **caractérisé en ce que**, à la place de visualiser un résultat ininterprétable lorsqu'au moins la mesure Doppler l'est, le diagnostic est dans ce cas basé sur la mesure du signal stéthoscopique, chaque diode du module (39) émettant dans la couleur spécifique correspondant, respectivement, à un diagnostic positif, un diagnostic négatif, ou un résultat ininterprétable, au cas où le signal stéthoscopique est ininterprétable, ou de dysfonctionnement de l'appareil, le diagnostic est alors basé sur le signal stéthoscopique sonore.

9. Appareil de dépistage et de dépistage et de diagnostic médical selon l'une quelconque des revendications précédentes, dans lequel un système d'enregistrement et de visualisation du signal vidéo Doppler ou stéthoscopique est prévu par liaison sans fil entre le circuit de traitement électronique (37) et un module de visualisation ou d'impression (50).

10. Appareil de dépistage et de diagnostic médical selon l'une quelconque des revendications précédentes, dans lequel des sorties périphériques (101, 42) sont prévues afin de permettre une liaison à un micro-ordinateur et en option à un casque auditif.

11. Appareil de dépistage et de diagnostic médical selon l'une quelconque des revendications précédentes, dans lequel, pour réaliser simplement la mise en service de la sonde notamment à l'aide d'un doigt (35), il est prévu un circuit électrique (29) de mise sous tension de la sonde ultrasonore (8), commandé par un actionneur (18, 28, 38) pouvant être monté sur le conduit de liaison (3, 33) ou sur le boîtier (100).

12. Appareil de dépistage et de diagnostic médical selon la revendication précédente, dans lequel l'actionneur est un interrupteur multifonctionnel qui sert également de commande sélective aux moyens de délivrance de diagnostics (29) stéthoscopique, Doppler ou croisé par les moyens de visualisation (31, 32, 39), aux moyens de déclenchement du diagnostic (30) à partir de mesures délivrées par le circuit de traitement (37) ou saisies à partir d'écoutes, ainsi qu'au système d'enregistrement et de visualisation à distance (50), la multifonction étant réalisée par des paliers différents, identifiés par une table de décision ou une logique de programmation des connexions des circuits en fonction du nombre de sollicitations de l'actionneur (18, 28, 38).

13. Appareil de dépistage et de diagnostic médical selon l'une quelconque des revendications précédentes, dans lequel une alimentation par pile ou par batterie rechargeable (23) est également prévue.

14. Appareil de dépistage et de diagnostic médical selon l'une quelconque des revendications précédentes, dans lequel le boîtier (100) forme le cornet acoustique (1) logeant la sonde ultrasonore (8), en particulier de manière centrée, et en ce que des moyens de contact (7) sont prévus pour s'interposer temporairement entre la sonde ultrasonore (8) et la membrane (2) du cornet acoustique (1), pour transmettre un signal Doppler au circuit de traitement (37) couplé au haut-parleur (34) qui émet le signal audio amplifié dans le cornet acoustique (1).

15. Appareil de dépistage et de diagnostic médical selon la revendication précédente, dans lequel les moyens d'interposition comportent un ballonnet (7) gonflable coiffant l'extrémité distale de la sonde (8) et un dispositif de gonflage (10) du ballonnet (7) par du liquide.

16. Appareil de dépistage et de diagnostic médical selon la revendication précédente, dans lequel le dispositif de gonflage comprend une tubulure (6) mettant le ballonnet (7) en communication avec une source de liquide, et des moyens (10) destinés à refouler du liquide de la source dans la tubulure (6).

17. Appareil de dépistage et de diagnostic médical selon l'une quelconque des revendications 14 à 16, dans lequel les moyens d'interposition (7) entre la sonde (8) et la membrane (2) sont commandés de l'extérieur du cornet (1) par le bouton actionneur (18).

18. Appareil de dépistage et de diagnostic médical selon l'une quelconque des revendications 14 à 17, dans lequel des moyens commandés de l'extérieur du cornet (1) et destinés à faire basculer la sonde (8) sont prévus en liaison avec le bouton actionneur (18).

19. Appareil de dépistage et de diagnostic médical selon l'une quelconque des revendications 14 à 18, dans lequel les moyens destinés à faire basculer la sonde (8) comportent au moins un câble (21), dont une extrémité est fixée à l'extrémité de la sonde (8), et des moyens (12) destinés à tirer l'autre extrémité du câble (21) et faire basculer l'extrémité de la sonde pour l'orienter vers la réponse sonore la plus perceptible à l'oreillette.

20. Appareil de dépistage et de diagnostic médical selon l'une quelconque des revendications 14 à 19, dans lequel il est prévu un circuit de mise sous tension (29) de la sonde ultrasonore (8) commandé par le bouton actionneur (18).

21. Appareil de dépistage et de diagnostic selon l'une des revendications précédentes, dans lequel il est prévu un circuit (30) d'enregistrement du signal Doppler commandé par le bouton actionneur (18).

22. Appareil de dépistage et de diagnostic selon l'une quelconque des revendications 14 à 21, dans lequel les boutons actionneurs forment un seul et même bouton, et il est prévu des moyens (11) destinés à maintenir le refoulement du liquide quand le bouton actionneur est relâché, les moyens de maintien comprenant un piston (10) en un matériau magnétique de refoulement du liquide, et une bobine (11) électromagnétique appliquant une force magnétique de maintien au piston (10).

23. Appareil de dépistage et de diagnostic médical selon l'une quelconque des revendications 1 à 13, dans lequel la sonde (8) est logée dans le boîtier (100) et hors du cornet (1'), le boîtier formant une tourelle sensiblement cylindrique.

24. Appareil de dépistage et de diagnostic médical selon l'une quelconque des revendications 1 à 13, dans lequel la sonde (8) est logée partiellement dans le boîtier (100) et partiellement hors du boîtier, la sonde traversant le boîtier (100) par une bague d'étanchéité (75) qui isole mécaniquement la sonde (8).

25. Appareil de dépistage et de diagnostic médical selon la revendication précédente, dans lequel le boîtier présente une partie inférieure (100b) cintrée dans sa partie centrale.

26. Appareil de dépistage et de diagnostic médical selon l'une quelconque des revendications 1 à 13, dans lequel la sonde (8) est hors du boîtier (100) qui se réduit à une partie supérieure (100a) de traitement de signal, la sonde (8) étant fixée le long du cornet (1).

27. Appareil de dépistage et de diagnostic médical selon la revendication 23, dans lequel la sonde (8) est inclinée vers l'axe central (X'X) du cornet d'un angle fixe choisi entre 30 et 70 degrés par rapport au plan de la membrane (2), de préférence entre 40 et 55 degrés, afin d'optimiser l'examen en faisant converger la réception des signaux ultrasonores et celle des signaux stéthoscopiques.

28. Appareil de dépistage et de diagnostic médical selon la revendication 23, dans lequel le boîtier (100) présente une forme de tourelle, sensiblement cylindrique et de section ovoïde, la tourelle est limitée par une face supérieure (Fs), au centre de laquelle émerge le conduit de liaison (3, 33), et par une face inférieure ouverte (Fi), où viennent se placer la membrane (2) du cornet (1') et l'extrémité (8a) de la sonde.

29. Appareil de dépistage et de diagnostic médical selon l'une quelconque des revendications 23 à 28, dans lequel la sonde est prolongée et il est prévu des moyens de délivrance (60, 6a, 6b) du produit semi-solide (61) forment une couche de liaison entre l'extrémité (8a) du prolongement (8b) de la sonde (8) et la peau du patient.

30. Appareil de dépistage et de diagnostic médical selon la revendication précédente, **caractérisé en ce qu'**il comprend un piston (36) qui commande le produit semi-solide et est accessible du boîtier (100), en particulier de la face supérieure (Fs), l'interrupteur (38) de mise en tension de la sonde 8 étant également disposé sur le boîtier.

31. Appareil de dépistage et de diagnostic médical selon la revendication précédente, **caractérisé en ce qu'**il comprend un réservoir (60) disposé dans le boîtier (100), le gel étant délivré à travers un tube souple (6a) par une buse d'éjection (6b) située en contact avec la face inférieure (Fi) de la tourelle (100) et la poussée du piston (36) permettant de doser la quantité adéquate de gel délivrée par la buse (6b).

32. Appareil de dépistage et de diagnostic médical selon l'une quelconque des revendications 23 à 31, dans lequel la sonde (8) est reliée à un haut-parleur (34), monté sur une face externe du cornet (1') via le circuit transducteur (37), le signal Doppler est converti par le circuit transducteur (37) pour fournir un signal audio par le haut-parleur (34), le son étant amplifié dans le cornet, propagé dans le conduit de liaison (3, 33) puis écouté au niveau des oreillettes (4).

33. Appareil de dépistage et de diagnostic médical selon la revendication précédente, dans lequel le logiciel d'interprétation qui commande le microprocesseur d'un micro-ordinateur à brancher sur une sortie (101) prévue sur le boîtier (100), comprend des moyens de saisie et de mémoire pour les résultats des écoutes stéthoscopique et/ou Doppler.

34. Appareil de dépistage et de diagnostic médical selon la revendication précédente, dans lequel, le micro-ordinateur est équipé d'un écran qui visualise le graphe du signal Doppler après que le signal Doppler a été converti par le circuit (37) et également transmis au micro-ordinateur et mémorisé sous forme de signal vidéo à travers la sortie (101).

35. Appareil de dépistage et de diagnostic médical selon la revendication 33 ou 34, dans lequel le logiciel fournit un diagnostic à partir des évaluations saisies et mémorisées, à l'aide du module d'affichage à au moins une diode électroluminescente (39, 39a), monté sur le boîtier (100) et couplé au circuit transducteur (37) pour visualiser l'interprétation.

36. Appareil de dépistage et de diagnostic médical selon l'une quelconque des revendications 23 à 35, dans lequel le circuit de transduction (37) convertit en signaux vidéo le signal stéthoscopique sonore reçu par le microphone selon la revendication 4 et le signal Doppler reçu par la sonde (8).

37. Appareil de dépistage et de diagnostic médical selon la revendication précédente, dans lequel les signaux vidéo sont transmis au micro-ordinateur par la sortie (101) et/ou vers un module de visualisation et d'impression (50), situé à distance.

38. Appareil de dépistage et de diagnostic selon la revendication 36 ou 37, dans lequel une antenne (41) est prévue pour émettre les signaux vidéo captés par le récepteur (51) du module de visualisation (50), puis traité dans un démodulateur (52) et dans un adaptateur de visualisation (53).

39. Appareil de dépistage et de diagnostic médical selon l'une quelconque des revendications 36 à 38, dans lequel une sortie casque (42) est également prévue afin de permettre une écoute stéthoscopique à partir du son capté par le microphone ou à partir du signal Doppler converti en signal audio par le circuit (37).

40. Appareil de dépistage et de diagnostic médical selon l'une quelconque des revendications 36 à 39, dans lequel les signaux vidéo, et éventuellement audio après saisie, sont transmis au microprocesseur (100) pour une évaluation et visualisés par l'écran du microprocesseur.

41. Appareil de dépistage et de diagnostic médical selon l'une quelconque des revendications 36 à 40, dans lequel le module d'affichage à diodes électroluminescentes (39, 39a) visualise une interprétation directe ou croisée à partir des signaux vidéo Doppler et stéthoscopique selon la revendication 7 ou 8.

## Claims

1. Medical screening and diagnostic apparatus using double detection of the stethoscopic and Doppler signals, comprising a sound transmission connecting line (3, 33) connected, at one end, to a housing (100) which at least partially forms an acoustic horn (1, 1') provided with a membrane (2) and, at the other end, to at least one earpiece (4) for listening to a stethoscopic signal coming from the horn, **characterised in that** the housing (100) is coupled to at least one ultrasound probe (8), designed to allow convergent reception of the ultrasonic and stethoscopic signals in order to simultaneously provide double detection, and connected to a transducer processing circuit (37) which is able to supply an audio signal from a Doppler signal by coupling the processing circuit (37) to a loudspeaker (34) in contact with the horn (1, 1') for stethoscope-type listening, and a video signal by coupling the processing circuit (37) to display means (31, 32, 39) for providing visual information.

2. Medical screening and diagnostic apparatus according to the previous claim, wherein means for delivering (60, 6a) and forming (6b) a film of semisolid product (61), particularly a gel, on the patient's skin are provided, in order to achieve intimate contact between the skin and the housing and channel the wave propagation.

3. Medical screening and diagnostic apparatus according to claim 1 or 2, wherein a loudspeaker (34) is provided which is disposed substantially adjacent to the acoustic horn (1, 1') so that the audio signal is amplified by the horn and renders the stethoscopic sound perceptible at the earpiece (4) through the connecting line (3, 33), in the same way as in a stethoscope.

4. Medical screening and diagnostic apparatus according to any one of claims 1 to 3, wherein a microphone (40) is provided, coupled to the horn (1, 1'), for picking up the stethoscopic sound signal and transmitting it in the form of an electrical signal to the processing circuit (37) and producing a video signal.

5. Medical screening and diagnostic apparatus according to any one of the preceding claims, wherein the display means are in the form of a liquid crystal screen (32) which allows graphic display of a stethoscopic and Doppler signal or in the form of a light-emitting diode module (39).

6. Medical screening and diagnostic apparatus according to any one of the preceding claims, **characterised in that** it comprises a microprocessor controlled by an interpretation algorithm and coupled to the processing circuit (37) to enable analysis and combination of the stethoscopic and/or Doppler measurements, supplied by the processing circuit (37) or picked up by listening through a stethoscope, and supplying a stethoscopic, Doppler and/or combined diagnosis.

7. Medical screening and diagnostic apparatus according to any one of the preceding claims, **characterised in that** it comprises a display module with three light-emitting diodes (39) which is mounted on the housing (100), which displays the interpretation and provides a diagnosis based on the measurement of the Doppler signal or a combined diagnosis based on the interpretation algorithm, giving preference to the Doppler diagnosis when the interpretations differ, each diode of the module (39) emitting a specific colour corresponding, respectively, to a positive diagnosis, a negative diagnosis or an uninterpretable result, in the event that at least the Doppler measurement is uninterpretable.

8. Medical diagnostic apparatus according to the preceding claim, **characterised in that**, instead of displaying an uninterpretable result when at least the Doppler measurement is uninterpretable, the diagnosis in this case is based on the measurement of the stethoscopic signal, each diode of the module (39) emitting the specific colour corresponding, respectively, to a positive diagnosis, a negative diagnosis or an uninterpretable result, in the event that the stethoscopic signal is uninterpretable, or in the event of malfunction of the apparatus, the diagnosis is then based on the stethoscopic sound signal.

9. Medical screening and diagnostic apparatus according to any one of the preceding claims, wherein a system for recording and displaying the Doppler or stethoscopic video signal is provided by a wireless connection between the electronic processing circuit (37) and a display or printing module (50).

10. Medical screening and diagnostic apparatus according to any one of the preceding claims, wherein peripheral outputs (101, 42) are provided to enable connection to a microcomputer and optionally to headphones.

11. Medical screening and diagnostic apparatus according to any one of the preceding claims, wherein, for simply operating the probe, particularly using a finger (35), an electric circuit (29) for supplying current to the ultrasound probe (8) is provided, which is controlled by an actuator (18, 28, 38) which can be mounted on the connecting line (3, 33) or on the housing (100).

12. Medical screening and diagnostic apparatus according to the preceding claim, wherein the actuator is a multifunctional switch which also acts as a selective control for the means for supplying stethoscopic, Doppler or mixed diagnoses (29) by the display means (31, 32, 39), the means for triggering the diagnosis (30) from measurements supplied by the processing circuit (37) or picked up by listening, and the remotely operated recording and display system (50), the multifunction being carried out by different stages identified by a decision table or programming logic for the connections of the circuits depending on the number of operations of the actuator (18, 28, 38).

13. Medical screening and diagnostic apparatus according to any one of the preceding claims, wherein the apparatus may also be powered by a normal or rechargeable battery (23).

14. Medical screening and diagnostic apparatus according to any one of the preceding claims, wherein the housing (100) forms the acoustic horn (1) which accommodates the ultrasound probe (8), particularly in centred manner, and in that contact means (7) are provided for temporary interposition between the ultrasound probe (8) and the membrane (2) of the acoustic horn (1), in order to transmit a Doppler signal to the processing circuit (37) coupled to the loudspeaker (34) which emits the audio signal amplified in the acoustic horn (1).

15. Medical screening and diagnostic apparatus according to the preceding claim, wherein the interposing means comprise an inflatable balloon (7) fitted to the distal end of the probe (8) and a device (10) for inflating the balloon (7) with liquid.

16. Medical screening and diagnostic apparatus according to the preceding claim, wherein the inflating device comprises a tube (6) which connects the balloon (7) to a source of liquid, and means (10) for displacing the liquid from the source into the tube (6).

17. Medical screening and diagnostic apparatus according to any one of claims 14 to 16, wherein the means (7) for interposing between the probe (8) and the membrane (2) are controlled from outside the horn (1) by the actuating knob (18).

18. Medical screening and diagnostic apparatus according to any one of claims 14 to 17, wherein means controlled from outside the horn (1) and adapted to swivel the probe (8) are provided in conjunction with the actuating knob (18).

19. Medical screening and diagnostic apparatus according to any one of claims 14 to 18, wherein the means for swivelling the probe (8) comprise at least one cable (21), one end of which is fixed to the end of the probe (8), and means (12) adapted to pull the other end of the cable (21) and swivel the end of the probe in order to orient it towards the sound response which is most audible in the earpiece.

20. Medical screening and diagnostic apparatus according to any one of claims 14 to 19, wherein a circuit (29) for supplying current to the ultrasound probe (8) is provided, controlled by the actuating knob (18).

21. Screening and diagnostic apparatus according to one of the preceding claims, wherein a circuit (30) for recording the Doppler signal is provided, controlled by the actuating knob (18).

22. Screening and diagnostic apparatus according to any one of claims 14 to 21, wherein the actuating knobs form one and the same knob, and means (11) are provided which are adapted to maintain the displacement of the liquid when the actuating knob is released, the maintaining means comprising a piston (10) made of a magnetic material for displacing the liquid, and an electromagnetic coil (11) applying a magnetic holding force to the piston (10).

23. Medical screening and diagnostic apparatus according to any one of claims 1 to 13, wherein the probe (8) is accommodated in the housing (100) and outside the horn (1'), the housing forming a substantially cylindrical turret.

24. Medical screening and diagnostic apparatus according to any one of claims 1 to 13, wherein the probe (8) is accommodated partially in the housing (100) and partially outside the housing, the probe passing through the housing (100) via a sealing ring (75) which mechanically isolates the probe (8).

25. Medical screening and diagnostic apparatus according to the preceding claim, wherein the housing has a lower part (100b) which is curved in its central part.

26. Medical screening and diagnostic apparatus according to any one of claims 1 to 13, wherein the probe (8) is outside the housing (100) which is reduced to an upper part (100a) for signal processing, the probe (8) being secured along the horn (1).

27. Medical screening and diagnostic apparatus according to claim 23, wherein the probe (8) is inclined towards the central axis (x'x) of the horn by a fixed angle selected between 30° and 70° relative to the plane of the membrane (2), preferably between 40° and 55°, in order to improve examination by converging the reception of the ultrasound signals with that of the stethoscopic signals.

28. Medical screening and diagnostic apparatus according to claim 23, wherein the housing (100) is in the form of a substantially cylindrical turret of ovoid cross-section, the turret is bounded by an upper surface (Fs), from the centre of which the connecting line (3, 33) emerges, and by an open lower surface (Fi), in which the membrane (2) of the horn (1') and the end (8a) of the probe are placed.

29. Medical screening and diagnostic apparatus according to any one of claims 23 to 28, wherein the probe is extended and delivery means (60, 6a, 6b) are provided for the semisolid product (61) forming a connecting layer between the end (8a) of the extension (8b) of the probe (8) and the patient's skin.

30. Medical screening and diagnostic apparatus according to the preceding claim, **characterised in that** it comprises a piston (36) which controls the semisolid product and is accessible from the housing (100), particularly the upper surface (Fs), the switch (38) for supplying current to the probe (8) also being arranged on the housing.

31. Medical screening and diagnostic apparatus according to the preceding claim, **characterised in that** it comprises a reservoir (60) arranged in the housing (100), the gel being supplied through a flexible tube (6a) by an ejector nozzle (6b) which is in contact with the lower surface (Fi) of the turret (100), and a pushing action on the piston (36) allowing the required amount of gel delivered through the nozzle (6b) to be measured out.

32. Medical screening and diagnostic apparatus according to any one of claims 23 to 31, wherein the probe (8) is connected to a speaker (34) mounted on an outer surface of the horn (1') via the transducer circuit (37), the Doppler signal is converted by the transducer circuit (37) to supply an audio signal through the loudspeaker (34), the sound being amplified in the horn, propagated into the connecting line (3, 33), then heard in the earpieces (4).

33. Medical screening and diagnostic apparatus according to the preceding claim, wherein the interpretation software which controls the microprocessor of a microcomputer to be plugged into an output (101) provided on the housing (100) comprises means for recording and storing the results of stethoscopic and/or Doppler listening.

34. Medical screening and diagnostic apparatus according to the preceding claim, wherein the microcomputer is fitted with a screen which displays the graph of the Doppler signal after the Doppler signal has been converted by the circuit (37) and also sent to the microcomputer and stored in the form of a video signal through the output (101).

35. Medical screening and diagnostic apparatus according to claim 33 or 34, wherein the software supplies a diagnosis from the evaluations recorded and stored, using the display module with at least one light emitting diode (39, 39a), mounted on the housing (100) and coupled to the transducer circuit (37) for displaying the interpretation.

36. Medical screening and diagnostic apparatus according to any one of claims 23 to 35, wherein the transduction circuit (37) converts, into video signals, the stethoscopic sound signal received by the microphone according to claim 4 and the Doppler signal received by the probe (8).

37. Medical screening and diagnostic apparatus according to the preceding claim, wherein the video signals are transmitted to the microcomputer through the output (101) and/or to a remotely located display and printing module (50).

38. Screening and diagnostic apparatus according to claim 36 or 37, wherein an aerial (41) is provided for transmitting the video signals picked up by the receiver (51) of the display module (50), then processed in a demodulator (52) and in a display adaptor (53).

39. Medical screening and diagnostic apparatus according to any one of claims 36 to 38, wherein a headset output (42) is also provided to allow stethoscopic listening from the sound picked up by the microphone or from the Doppler signal converted into an audio signal by the circuit (37).

40. Medical screening and diagnostic apparatus according to any one of claims 36 to 39, wherein the video signals and possibly audio signals after they have been picked up are transmitted to the microprocessor (100) for evaluation and displayed on the screen of the microprocessor.

41. Medical screening and diagnostic apparatus according to any one of claims 36 to 40, wherein the display module with light emitting diodes (39, 39a) displays a direct or mixed interpretation from the Doppler and stethoscopic video signals according to claim 7 or 8.

## Patentansprüche

1. Vorrichtung zur Untersuchung und zur medizinischen Diagnose durch Doppelerfassung von Stethoskop- und Dopplersignalen, die eine Verbindungsleitung (3, 33) zur Schallübertragung umfasst, die an einem Ende mit einem Gehäuse (100), das wenigstens teilweise einen Schalltrichter (1, 1') bildet, der eine Membran (2) aufweist, und an dem anderen Ende mit wenigstens einer Hörolive (4) für ein von dem Schalltrichter kommendes Signal, verbunden ist, **dadurch gekennzeichnet, dass** das Gehäuse (100) mit wenigstens einer Ultraschallsonde (8) gekoppelt ist, die derart angeordnet ist, dass eine Empfangszusammenführung der Ultraschall- und Stethoskopiesignale ermöglicht ist, um zeitgleich eine Doppelerfassung zu liefern, und die mit einer Messwandlerschaltung (37) verbunden ist, die durch Kopplung der Messwandlerschaltung (37) mit einem sich für ein stethoskopisches Abhorchen mit dem Schalltrichter (1, 1') in Kontakt befindlichen Lautsprecher (34) aus einem Dopplersignal ein Audiosignal und durch Kopplung der Messwandlerschaltung (37) mit Visualisierungsmitteln (31, 32, 39) zur Bereitstellung visueller Informationen ein Videosignal liefern kann.

2. Vorrichtung zur Untersuchung und zur medizinischen Diagnose nach dem vorherigen Anspruch, in der Mittel zum Abgeben (60, 6a) und zur Bildung (6b) einer dünnen Schicht (61) aus einem halbfesten Produkt, insbesondere ein Gel, auf der Haut eines Patienten vorgesehen sind, um einen engen Haut-/Gehäuse-Kontakt herzustellen und die Ausbreitung von Wellen zu kanalisieren.

3. Vorrichtung zur Untersuchung und zur medizinischen Diagnose nach Anspruch 1 oder 2, in der ein Lautsprecher (34) vorgesehen ist, der im Wesentlichen an den Schalltrichter (1, 1') anliegend angeordnet ist, so dass das Audiosignal durch den Trichter verstärkt wird und das stethoskopische Abhorchen über die Verbindungsleitung (3, 33) auf die gleiche Weise wie in einem Stethoskop bei der Olive (4) möglich ist.

4. Vorrichtung zur Untersuchung und zur medizinischen Diagnose nach einem der Ansprüche 1 bis 3, in der ein Mikrofon (40) vorgesehen ist, das mit dem Trichter (1, 1') verbunden ist, um das stethoskopische Schallsignal aufzunehmen und es in Form eines elektrischen Signals an die Messwandlerschaltung (37) zu übertragen und ein Videosignal zu erzeugen.

5. Vorrichtung zur Untersuchung und zur medizinischen Diagnose nach einem der vorherigen Ansprüche, in der die Visualisierungsmittel in Form eines Flüssigkristallbildschirms (32), wodurch die grafische Visualisierung eines Stethoskopie- und Dopplersignals ermöglicht wird, oder in Form eines Moduls (39) mit Leuchtdioden gegeben sind.

6. Vorrichtung zur Untersuchung und zur medizinischen Diagnose nach einem der vorherigen Ansprüche, **dadurch gekennzeichnet, dass** sie einen Mikroprozessor umfasst, der von einem Interpretationsalgorithmus gesteuert wird und mit der Messwandlerschaltung (37) gekoppelt ist, um eine Analyse und eine Kombination der Stethoskopie- und/oder Dopplermessungen zu ermöglichen, die von der Messwandlerschaltung (37) geliefert oder durch stethoskopisches Abhorchen erfasst werden, und um eine stethoskopische, Doppler- und/oder gemischte Diagnose zu liefern.

7. Vorrichtung zur Untersuchung und zur medizinischen Diagnose nach einem der vorherigen Ansprüche, **dadurch gekennzeichnet, dass** sie ein Anzeigemodul (39) mit drei Leuchtdioden umfasst, das auf dem Gehäuse (100) befestigt ist und die Interpretation visualisiert und eine Diagnose auf der Grundlage der Messung des Dopplersignals oder eine Mischdiagnose auf der Grundlage des Interpretationsalgorithmus liefert, wobei die Dopplerdiagnose Vorrang hat, wenn die Interpretationen streuen, wobei jede Diode des Moduls (39) eine bestimmte Farbe aussendet, die einer positiven Diagnose, einer negativen Diagnose bzw. - in einem Fall, in dem wenigstens die Dopplermessung nicht interpretierbar ist - einem nicht interpretierbaren Ergebnis entspricht.

8. Vorrichtung zur Untersuchung und zur medizinischen Diagnose, **dadurch gekennzeichnet, dass**, statt ein nicht interpretierbares Ergebnis zu visualisieren, wenn wenigstens die Dopplermessung interpretierbar ist, die Diagnose in diesem Fall auf der Messung des stethoskopischen Signals basiert, wobei jede Diode des Moduls (39) in der bestimmten Farbe aussendet, die einer positiven Diagnose, einer negativen Diagnose oder - in dem Fall, in dem das stethoskopische Signal nicht interpretierbar ist, oder einer Fehlfunktion der Vorrichtung - entspricht, die Diagnose basiert dann auf dem stethoskopischen Schallsignal.

9. Vorrichtung zur Untersuchung und zur medizinischen Diagnose nach einem der vorherigen Ansprüche, in dem ein Aufzeichnungs- und Visualisierungssytem für das Videodoppler- oder Stethoskopiesignal über eine drahtlose Verbindung zwischen der elektronischen Messwandlerschaltung (37) und einem Visualisierungs- oder Druckmodul (50) vorgesehen ist.

10. Vorrichtung zur Untersuchung und zur medizinischen Diagnose nach einem der vorherigen Ansprüche, in dem Peripherieausgänge (101, 42) vorgesehen sind, um eine Verbindung mit einem Mikrocomputer oder alternativ mit eine Kopfhörer zu ermöglichen.

11. Vorrichtung zur Untersuchung und zur medizinischen Diagnose nach einem der vorherigen Ansprüche, in der, um eine einfache Handhabung der Sonde, insbesondere mit Hilfe eines Fingers (35), zu bewerkstelligen, eine elektrische Schaltung (29) zum Unter-Spannung-Setzen der Ultraschallsonde (8) vorgesehen ist, die von einem Betätigungselement (18, 28, 38) angesteuert wird, das auf der Verbindungsleitung (3, 33) oder auf dem Gehäuse (100) angebracht sein kann.

12. Vorrichtung zur Untersuchung und zur medizinischen Diagnose nach einem der vorherigen Ansprüche, in der das Betätigungselement ein Multifunktionsschalter ist, der gleichfalls als Steuerungselement zur Auswahl zwischen Mitteln (29) zur stethoskopischen, Doppler- oder gemischten Diagnose-Erstellung durch die Visualisierungsmittel (31, 32, 39), Mitteln (30) zur Auslösung der Diagnose ausgehend von Messungen, die von der Messwandlerschaltung (37) geliefert oder durch Abhorchen erfasst werden, sowie dem System (50) zur Fernaufzeichnung und -visualisierung dient, wobei die Multifunktion durch unterschiedliche Phasen verwirklicht ist, die durch eine Entscheidungstabelle oder einer Logik der Programmierung von Verbindungen der Schaltungen als Funktion der Anzahl von Betätigungen des Betätigungselements (18, 28, 38) bestimmt sind.

13. Vorrichtung zur Untersuchung und zur medizinischen Diagnose nach einem der vorherigen Ansprüche, in der ferner eine Versorgung durch eine Batterie oder einen Akkumulator (23) vorgesehen ist.

14. Vorrichtung zur Untersuchung und zur medizinischen Diagnose nach einem der vorherigen Ansprüche, **dadurch gekennzeichnet, dass** das Gehäuse (100) den Schalltrichter (1) bildet, in dem die Ultraschallsonde (8) insbesondere mittig aufgenommen ist, und **dadurch**, dass Kontaktmittel (7) vorgesehen sind, die vorübergehend zwischen der Ultraschallsonde (8) und der Membran (2) des Schalltrichters (1) zwischengefügt sind, um ein Dopplersignal an die Messwandlerschaltung (37) zu übertragen, die mit dem Lautsprecher (34) gekoppelt ist, welcher das in dem Schalltrichter (1) verstärkte Audiosignal aussendet.

15. Vorrichtung zur Untersuchung und zur medizinischen Diagnose nach einem der vorherigen Ansprüche, in der die Zwischenfügungsmittel einen aufblähbaren Ballon (7), der das entfernte Ende der Sonde (8) bedeckt, und eine Mittel (10) zum Aufblähen des Ballons (7) mittels Flüssigkeit umfassen.

16. Vorrichtung zur Untersuchung und zur medizinischen Diagnose nach dem vorherigen Anspruch, in der die Aufblähvorrichtung ein Rohrstutzen (6), welcher eine Verbindung zwischen dem Ballon (7) und einer Flüssigkeitsquelle erstellt, und Mittel (10), die dazu dienen, die Flüssigkeit der Quelle in den Rohrstutzen (6) zu drängen, umfasst.

17. Vorrichtung zur Untersuchung und zur medizinischen Diagnose nach einem der Ansprüche 14 bis 16, in der die Mittel zur Zwischenfügung (7) zwischen die Sonde (8) und die Membran (2) von außerhalb des Trichters
(1) durch einen Betätigungsknopf (18) betätigt werden.

18. Vorrichtung zur Untersuchung und zur medizinischen Diagnose nach einem der Ansprüche 14 bis 17, in der Mittel, die von außerhalb des Trichters (1) betätigt werden und dazu dienen, die Sonde (8) zu kippen, in Verbindung mit dem Betätigungsknopf (18) vorgesehen sind.

19. Vorrichtung zur Untersuchung und zur medizinischen Diagnose nach einem der Ansprüche 14 bis 18, in der die Mittel, die zum Kippen der Sonde (8) vorgesehen sind, wenigstens ein Kabel (21), dessen eines Ende an dem Ende der Sonde (8) befestigt ist, und Mittel (12), die dazu dienen, das weitere Ende des Kabels (21) zu ziehen und das Ende der Sonde zu kippen, um sie in Richtung der an der Olive am deutlichsten wahrnehmbaren Schallantwort zu orientieren, umfassen.

20. Vorrichtung zur Untersuchung und zur medizinischen Diagnose nach einem der Ansprüche 14 bis 19, in der eine Schaltung (29) zum Unter-Spannung-Setzen der durch den Betätigungsknopf (18) betätigten Ultraschallsonde (8) vorgesehen ist.

21. Vorrichtung zur Untersuchung und zur medizinischen Diagnose nach einem der vorherigen Ansprüche, in der eine Schaltung (30) zum Aufzeichnen des Dopplersignals vorgesehen ist, die von dem Betätigungsknopf (18) betätigt wird.

22. Vorrichtung zur Untersuchung und zur medizinischen Diagnose nach einem der Ansprüche 14 bis 21, in der die Betätigungsknöpfe ein und denselben Knopf bilden, und Mittel (11) vorgesehen sind, die dazu dienen, den Druck der Flüssigkeit aufrecht zu erhalten, wenn der Betätigungsknopf losgelassen wird, wobei die Mittel zur Aufrechterhaltung einen Kolben (10) aus einem magnetischen Material zur Druckbeaufschlagung der Flüssigkeit und eine elektromagnetische Spule (11), die eine magnetische Kraft zum Halten des Kolbens (10) ausübt, umfassen.

23. Vorrichtung zur Untersuchung und zur medizinischen Diagnose nach einem der Ansprüche 1 bis 13, in der die Sonde (8) in dem Gehäuse (100) und außerhalb des Schalltrichters (1') angeordnet ist, wobei das Gehäuse einen im Wesentlichen zylindrischen Überbau bildet.

24. Vorrichtung zur Untersuchung und zur medizinischen Diagnose nach einem der Ansprüche 1 bis 13, in der die Sonde (8) teilweise in dem Gehäuse (100) und teilweise außerhalb des Gehäuses angeordnet ist, wobei die Sonde das Gehäuse (100) durch einen Dichtring (75) durchdringt, der die Sonde (8) mechanisch isoliert.

25. Vorrichtung zur Untersuchung und zur medizinischen Diagnose nach dem vorherigen Anspruch, in der das Gehäuse einen unteren Teil (100b) aufweist, der in seinem mittleren Teil gewölbt ist.

26. Vorrichtung zur Untersuchung und zur medizinischen Diagnose nach einem der Ansprüche 1 bis 13, in der die Sonde (8) außerhalb des Gehäuses (100) ist, welches sich auf einen oberen Signalverarbeitungsteil (100a) begrenzt, wobei die Sonde (8) entlang des Schalltrichters (1) befestigt ist.

27. Vorrichtung zur Untersuchung und zur medizinischen Diagnose nach Anspruch 23, in der die Sonde (8) in Richtung der Mittenachse (X'X) des Trichters um einen festen Winkel, der zwischen 30 und 79 Grad, vorzugsweise zwischen 40 und 45 Grad, gewählt ist, bezüglich der Ebene der Membran (2) geneigt ist, um durch Zusammenführen des Empfangs der Ultraschallsignale und des der Stethoskopiesignale die Untersuchung zu optimieren.

28. Vorrichtung zur Untersuchung und zur medizinischen Diagnose nach Anspruch 23, in der das Gehäuse (100) die Form eines im Wesentlichen zylindrischen Überbaus mit ovalem Querschnitt aufweist, der Überbau von einer oberen Oberfläche (Fs), in deren Mitte das Verbindungsrohr (3, 33) herausragt, und von einer unteren, offenen Oberfläche (Fi), wo die Membran (2) des Trichters (1) und das Ende (8) der Sonde anzuordnen ist, begrenzt ist.

29. Vorrichtung zur Untersuchung und zur medizinischen Diagnose nach einem der Ansprüche 23 bis 28, in der die Sonde verlängert ist, und die Mittel (60, 6a, 6b) zur Abgabe des halbfesten Produkts (61) umfasst, das eine Verbindungsschicht zwischen dem Ende (8a) der Verlängerung (8b) der Sonde (8) und der Haut des Patienten bildet.

30. Vorrichtung zur Untersuchung und zur medizinischen Diagnose nach dem vorherigen Anspruch, **dadurch gekennzeichnet, dass** sie einen Kolben (36) umfasst, der das halbfeste Produkt vorantreibt und von dem Gehäuse (100) aus, insbesondere von der oberen Oberfläche (Fs), zugänglich ist, wobei der Schalter (38) zum Unter-Spannung-Setzen der Sonde (8) ebenfalls auf dem Gehäuse angeordnet ist.

31. Vorrichtung zur Untersuchung und zur medizinischen Diagnose nach dem vorherigen Anspruch, **dadurch gekennzeichnet, dass** sie einen Behälter (60) umfasst, der in dem Gehäuse (100) angeordnet ist, wobei das Gel über ein biegsames Rohr (6a) durch eine Spritzdüse (6b) abgegeben wird, die sich in Kontakt mit der unteren Oberfläche (Fi) des Überbaus (100) befindet, und wobei die Hubkraft des Kolbens (36) eine Dosierung der angemessenen Menge an Gel, das von der Düse (6b) abgegeben wird, erlaubt.

32. Vorrichtung zur Untersuchung und zur medizinischen Diagnose nach einem der Ansprüche 23 bis 31, in der die Sonde (8) über die Messwandlerschaltung (37) mit einem Lautsprecher (34), der auf einer äußeren Oberfläche des Trichters montiert ist, verbunden ist (1') und das Doppler-Signal durch die Messwandlerschaltung (37) umgewandelt wird, um über den Lautsprecher (34) ein Audiosignal zu liefern, wobei der Schall in dem Trichter verstärkt, in der Verbindungsleitung (3, 33) weitergeleitet und schließlich bei den Oliven (4) wahrgenommen wird.

33. Vorrichtung zur Untersuchung und zur medizinischen Diagnose nach dem vorherigen Anspruch, in der die Interpretationssoftware, die den Mikroprozessor des Mikrocomputers anweist, eine Verbindung zu einem Ausgang (101), der auf dem Gehäuse (100) vorgesehen ist, herzustellen, Erfassungs- und Speichermittel für die Ergebnisse der Stethoskopie- und/oder Dopplerabhorchung umfasst.

34. Vorrichtung zur Untersuchung und zur medizinischen Diagnose nach dem vorherigen Anspruch, in der der Mikrocomputer mit einem Bildschirm ausgestattet ist, der die Kennlinie des Dopplersignals visualisiert, nachdem das Doppler-Signal durch die Schaltung (37) umgewandelt, ebenfalls über den Ausgang (101) dem Mikrocomputer zugeführt und in Form eines Videosignals gespeichert wurde.

35. Vorrichtung zur Untersuchung und zur medizinischen Diagnose nach Anspruch 33 oder 34, in der die Software eine Diagnose auf der Grundlage der erfassten und gespeicherten Bewertungen mit Hilfe des Anzeigemoduls mit wenigstens einer Leuchtdiode (39, 39a), das auf dem Gehäuse (100) befestigt und zur Visualisierung der Interpretation mit der Messwandlerschaltung (37) gekoppelt ist, liefert.

36. Vorrichtung zur Untersuchung und zur medizinischen Diagnose nach einem der Ansprüche 23 bis 35, in der die Messwandlerschaltung (37) das von dem Mikrofon gemäß Anspruch 4 empfangene Stethoskopieschallsignal und das von der Sonde (8) empfangene Doppler-Signal in Videosignale umwandelt.

37. Vorrichtung zur Untersuchung und zur medizinischen Diagnose nach dem vorherigen Anspruch, in der die Videosignale über den Ausgang (101) zu dem Mikrocomputer und/oder zu einem beabstandeten Visualisierungs- und Druckmodul (50) übertragen werden.

38. Vorrichtung zur Untersuchung und zur medizinischen Diagnose nach dem Anspruch 36 oder 37, in der eine Antenne (41) vorgesehen ist, um von dem Empfänger (51) des Visualisierungsmoduls (50) empfangene und anschließend in einem Demodulator (52) und einem Visualisierungsadapter verarbeitete Videosignale auszusenden.

39. Vorrichtung zur Untersuchung und zur medizinischen Diagnose nach einem der Ansprüche 36 bis 38, in der ferner ein Kopfhörerausgang (42) vorgesehen ist, um aus dem über das Mikrofon empfangenen Schall oder aus dem durch die Schaltung (37) in ein Audiosignal umgewandelten Dopplersignal einen stethoskopischen Abhorchen zu ermöglichen.

40. Vorrichtung zur Untersuchung und zur medizinischen Diagnose nach einem der Ansprüche 36 bis 39, in der die Videosignale und schließlich nach der Erfassung die Audiosignale für eine Bewertung an den Mikroprozessor (100) gesendet und mittels des Bildschirms des Mikroprozessors visualisiert werden.

41. Vorrichtung zur Untersuchung und zur medizinischen Diagnose nach einem der Ansprüche 36 bis 40, in der das Anzeigemodul mit Leuchtdioden (39, 39a) eine direkte oder gemischte Interpretation aus den Doppler- oder Stethoskopievideosignalen gemäß Anspruch 7 oder 8 visualisiert.
